# EUROPÄISCHE PATENTSCHRIFT

(11) **EP 2 289 824 B1**
(45) Veröffentlichungstag und Bekanntmachung des Hinweises auf die Patenterteilung: **09.05.2012**
(21) Anmeldenummer: 10172730.3
(22) Anmeldetag: 13.08.2010
(51) Int. Cl.: B65G 25/08, B65G 65/44

(54) **Beschicker für Biomasse**
Feeder for biomass
Dispositif d'alimentation pour biomasse

(30) Priorität: 26.08.2009 DE 202009011482 U
(43) Veröffentlichungstag der Anmeldung: 02.03.2011
(73) Patentinhaber: Schmack Biogas GmbH, 92421 Schwandorf (DE)
(72) Erfinder: Hutzler, Norbert, 92724, Trabitz (DE); Seidl, Armin, 92421, Schwandorf (DE); Wimmer, Alfons, 83339, Chieming (DE); Meyer, Stefan, 92331, Parsberg (DE)
(74) Vertreter: Graf Glück Habersack Kritzenberger

(56) Entgegenhaltungen:
- DE-A1- 4 008 287
- DE-A1- 19 816 552

## Beschreibung

### Technisches Gebiet

Die Erfindung betrifft einen Beschicker für Biogasanlagen oder andere Anlagen, die mit Biomasse betrieben werden.

### Stand der Technik

Die Fördereinrichtung ist geeignet für den Transport aller Arten von Biomasse, beispielsweise fester Biomasse, die auch als Gemisch fester und flüssiger Anteile vorliegen kann. Beispiele für geeignete Fördergüter sind Mais- oder Grassilage, Gülle, Getreide, organische Abfälle, Klärschlamm, Holzhackschnitzel und/oder Holzpellets.

Bekannt sind Fördereinrichtungen mit einer Vielzahl parallel zueinander und versetzt angeordneter Schieber, die fixiert an bewegbaren Mittelachsen hin und her bewegt werden. Dadurch wird die Biomasse am Boden innerhalb des Beschickers und zum Auslass des Beschickers hin bewegt.

Im Betrieb werden die Schieber von einer Ausgangsposition durch die Bewegung der Mittelachse eine bestimmte Strecke, die im Wesentlichen dem Abstand zweier Bodenkanten entspricht, geschoben. An der Kante des Beschickerbodens wird die Biomasse entweder direkt in die Biogasanlage eingebracht oder gelangt über eine weitere Fördereinrichtung wie z.B. eine Förderschnecke in die Biogasanlage.

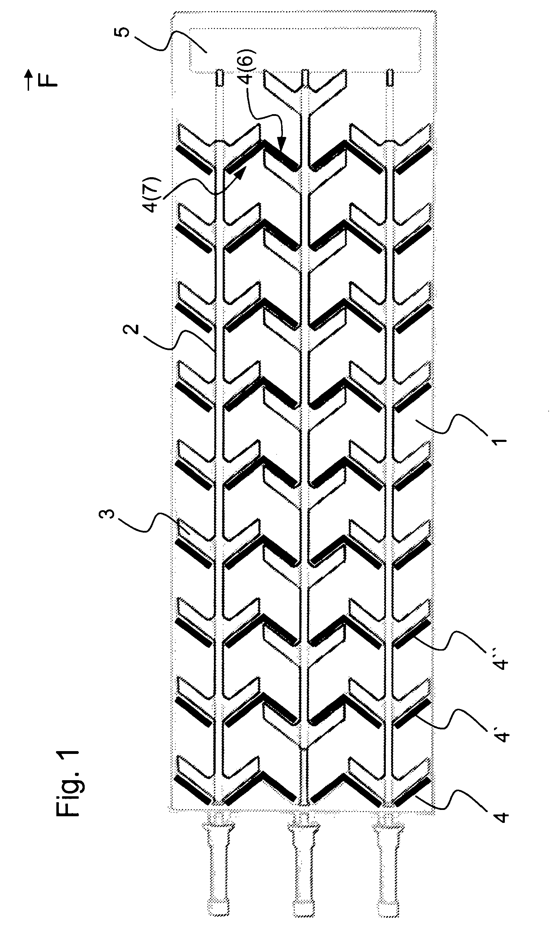

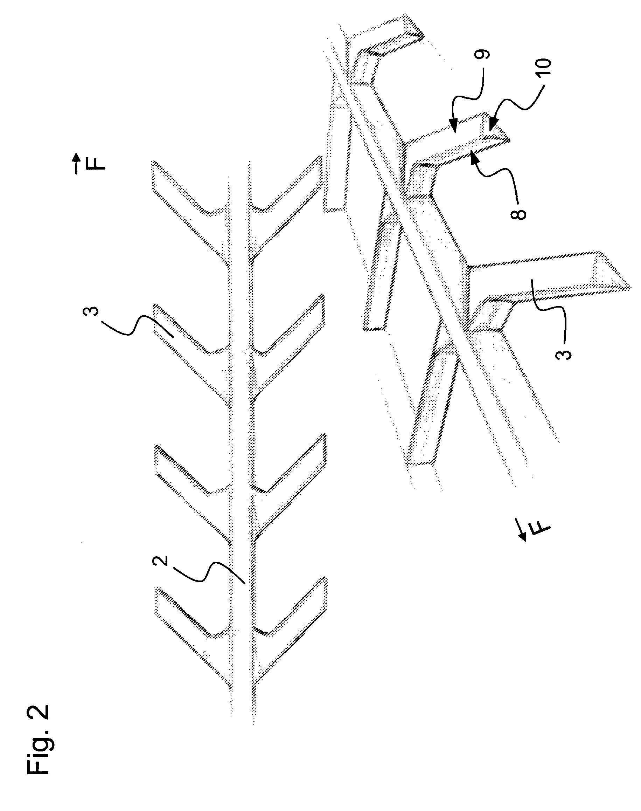

Aus der DE 10 2008 020244 A1 ist ein Beschicker für Biogasanlagen bekannt, wobei eine Fördereinrichtung, mit der die Rohstoffe in den Fermenter der Biogasanlage eingebracht werden, in den Figuren 1 und 2 zu erkennen ist.

Die Fördereinrichtung umfasst drei Mittelachsen die über geeignete Antriebe außerhalb des Beschickers hin- und her bewegt werden. An den Mittelachsen sind versetzt, senkrecht zur Mittelachse und parallel zueinander die Schieber angeordnet, die die Biomasse bewegen.

Die DE 20 2004 009 962 U1 und EP 1 609 355 A1 beschreiben einen Lagerbehälter für Biomasse mit einem Förderelement, dessen Förderarme im rechten Winkel seitlich abstehen und auf Kunststoffplatten gleiten, die mit Fixierleisten am Boden befestigt sind.

Die DE 20 2005 017 357 U1 und EP 1 783 069 A1 lehren einen befahrbaren Aufnahmebehälter aus Beton mit Schubbodenförderern, bei denen die Förderarme beidseitig in einem 90° Winkel angeordnet sind und der Aufnahmebehälter inklusive Fördereinrichtung auf Wägezellen zur automatischen Datenerfassung ruhen.

Aus den US-Patentschriften US 3,923,149 und US 4,363,586 sind ebenfalls Förderanlagen für geschredderte Biomasse bzw. Schüttgut bekannt, die das Transportgut mit Hilfe von leiterförmig aufgebauten Transporteinheiten bewegen.

Nachteilig an diesem Stand der Technik ist, dass durch die Schieber zuviel Biomasse wieder zurückgenommen wird.

Terbrack schlägt im Stand der Technik (DE 20 2007 009 040 U1) für einen Schubboden eine Schubstange mit einem Schieber mit mehrteiliger Schubklappe vor, bei der die beweglichen Flügel beim Vorschub einen Winkel nahe 90° einnehmen, während sie beim Rückzug an der Schubstange anliegen und einen Winkel nahe 0° annehmen. Diese spezielle Ausführung eines Schiebers soll insgesamt den Gleitwiderstand verringern.

Nachteilig an dieser Erfindung sind einerseits die hohen Herstellungskosten für eine derartige Fördereinrichtung bestehend aus mehreren Bauteilen sowie auch die hohe technische Anfälligkeit für derartige bewegliche Schubklappen. Vor allem für das hier zu bewegende Transportgut, das Substrat für Biogasanlagen, das auch gröbere, feste und/oder faserartige Bestandteile wie Stroh oder Ganzpflanzensilage aufweisen kann, erscheint eine Ausgestaltung der Schieber nach der Lehre von Terbrack nicht geeignet, da sich die beweglichen Teile verhaken können oder der Belastung durch größere Stücke nicht standhalten und brechen.

Schließlich ist aus der DE 40 08 287 A1 ein Beschicker gemäß dem Oberbegriff des Anspruchs 1 in einem Aufbewahrungsbehälter für kompostierbares Material bekannt. Der Behälter umfasst eine Schubboden-Förderanlage und einen an einem bodenseitigen Auslass des Behälters angeordneten Schneckförderer. Der Förderer weist eine bewegbare Mittelachse auf, wobei an der Mittelachse eine Anzahl feststehender Schieber in einem Winkel von 90° so angeordnet sind, dass jeder Schieber durch die Bewegung der Mittelachse aus der Anfangsposition an einer ersten Bodenkante heraus bis zu einer Endposition an der nächsten Bodenkante bewegt wird.

Der Schneckförderer der DE 40 08 287 A1 transportiert zwar das Fördergut in der Förderrichtung in ausreichendem Maß, beim Rückzug der Förderarme wird aber relativ viel Material mit zurückgenommen. Der Rückzug der Förderarme erfordert daher einen relativ hohen Kraftaufwand und führt zu einem hohen Verschleiß der Bauteile der Fördereinrichtung.

### Darstellung der Erfindung

Aufgabe der vorliegenden Erfindung ist es daher, einen Beschicker für Biogasanlagen zu schaffen, die gegenüber der zu bewegenden Biomasse verschleißfest ist und die, die fest und/oder als Gemisch aus fest und flüssig vorliegende Biomasse mit verbesserter Effizienz zur Kante des Bodens des Beschickers bewegt.

Dabei geht es - im Vergleich zum Stand der Technik - nur teilweise um den Transport größerer Mengen, im Wesentlichen geht es um weniger Biomasserücknahme, also im Endeffekt um eine höhere Effizienz im Vorschub und weniger Kraftaufwand und Verschleiß für die Anlage.

So besteht der Bedarf eine Fördereinrichtung zu schaffen, mit der Biomasse effizienter in Förderrichtung bewegt werden kann.

Allgemeine Erkenntnis der Erfindung ist, dass es ein Nachteil von Förderarmen, die sich im rechten Winkel zur Förderrichtung befinden, ist, dass sie zwar das Fördergut in der Förderrichtung gut transportieren, beim Rückzug der Förderarme jedoch auch wieder viel Material mit zurücknehmen. Der Rückzug der Förderarme erfordert einen relativ hohen Kraftaufwand und führt zu einem hohen Verschleiß der Bauteile der Fördereinrichtung.

Die Lösung der Aufgabe und der Gegenstand der Erfindung werden in den Ansprüchen definiert.

Gegenstand der vorliegenden Erfindung ist demgemäß ein Beschicker für Biogasanlagen oder andere Anlagen, die mit Biomasse betrieben werden, umfassend eine Fördereinrichtung, wobei die Fördereinrichtung eine oder mehrere bewegbare Mittelachsen am Boden des Beschickers umfasst. An jeder Mittelachse ist eine Anzahl feststehender Schieber so angeordnet ist, dass jeder Schieber durch die Bewegung der Mittelachse aus der Anfangsposition an einer ersten Bodenkante heraus bis zu einer Endposition an der nächsten Bodenkante bewegt wird. Die Schieber sind feststehend und symmetrisch auf einander gegenüberliegenden Seiten der Mittelachse angeordnet und schließen in Richtung eines Fördergutauslasses einen Winkel in einem Bereich von 20° bis 80° mit der Mittelachse ein.

Es handelt sich um einen Beschicker für Biomasse, insbesondere von fester Biomasse, die auch als Gemisch fester und flüssiger Anteile vorliegen kann, wobei eine oder mehrere bewegbare Mittelachsen am Boden des Beschickers vorgesehen sind.

An jeder Mittelachse sind eine Anzahl feststehender Schieber in einem Winkel in einem Bereich von 20° bis 80° direkt an der Mittelachse oder über Aufnahmevorrichtungen an der Mittelachse so angeordnet, dass der Schieber durch die Bewegung der Mittelachse aus der Anfangsposition heraus bis zu einer Bodenkante oder Auslasskante bewegt wird, an der der Schieber stoppt und dadurch die Biomasse über die Bodenkante drückt. Beispielsweise stoßen die Schieber nicht wirklich an, sondern stoppen vor den Bodenkanten. Es bleibt also gegebenenfalls auch ein kleiner Spalt. Dabei sind "Widerlager", die die Mittelachsen stoppen, nicht erforderlich, da der Vorschub der Mittelachsen durch den Hub des Hydraulikzylinders begrenzt wird.

Die eingebauten Bodenkanten haben in der vorliegenden Erfindung die Funktion von Substratrückhalteleisten, die das Zurückrutschen des Fördergutes bei der Bewegung der Mittelachsen entgegen der Förderrichtung vermindern. Wichtig ist dies insbesondere, wenn der Boden der Fördereinrichtung aus einem reibungsarmen Material wie Kunststoff oder Metall besteht.

Als "Kunststoff" wird vorliegend jeder synthetisch herstellbare zwischen + 100°C und -50°C feste Werkstoff bezeichnet, der organische oder metallorganische oder hybride Verbindungen, allein oder als Gemisch verschiedener Einzelverbindungen vorliegend, enthält. Als "Metall" wird hier jedes Metall oder jede Metallverbindung bezeichnet sowie auch Legierungen und Gemische von Legierungen oder mit Metallen. Mischungen von Metallen und/oder Legierungen mit Kunststoffen können wiederum unter den Begriff "Kunststoff" fallen.

Die Schieber, die als Förderarme dienen, sind symmetrisch um die Mittelachse angeordnet und schließen mit dieser einen Winkel in einem Bereich von 20° bis 80° ein. In einer besonders bevorzugten Ausführungsform schließen die Schieber mit der Mittelachse einen Winkel von 30° bis 60°, ganz besonders bevorzugt einen Winkel von 45° ein. Die Schieber sind in der Regel aus Metall gefertigt, insbesondere aus Stahl oder Edelstahl. Je nach Biomasse können die Schieber auch aus anderen Materialien sein.

Im Betrieb wird der Schieber dann zwischen der Ausgangsposition und der Endposition durch die entsprechende Bewegung der Mittelachse hin und hergefahren. Die Strecke des Vorschubs bzw. Rückzugs wird durch die technischen Gegebenheiten des Antriebs der beweglichen Mittelachsen bestimmt, der vorzugsweise hydraulisch oder pneumatisch erfolgt, also z.B. über den Hub eines Hydraulikzylinders. In einer bevorzugten Ausführungsform sind die Bodenkanten in genau dem Abstand angeordnet, dass sie sowohl die Ausgangs- als auch die Endposition der Schieber begrenzen.

Vorzugsweise verfügt jede Mittelachse über einen eigenen Antrieb, mit dem sie über eine Kolbenstange fest verbunden ist. Durch die entsprechende Positionierung und technische Ausgestaltung des Antriebs kann die erfindungsgemäße Fördereinrichtung sowohl als Schubboden wie auch als Zugboden betrieben werden. Der Antrieb der beweglichen Mittelachsen erfolgt je nach Anwendung und Beschaffenheit des Fördergutes unterschiedlich. Eine bevorzugte Antriebsform ist, dass alle Mittelachsen gleichzeitig aus der Ausgangs- in die Endposition bewegt werden, anschließend aber nacheinander in die Ausgangsstellung zurückgefahren werden. Eine weitere bevorzugte Antriebsform ist, dass nebeneinander befindliche Mittelachsen jeweils gegengleich bewegt werden, also jeweils jede zweite Mittelachse in Förderrichtung während die dazwischen liegenden Mittelachsen entgegen der Förderrichtung bewegt werden. Weitere Antriebformen können sich ebenfalls als vorteilhaft erweisen.

Nach einer vorteilhaften Ausführungsform ist die Bodenkante im Beschicker der Form nach den Schiebern angepasst, insbesondere sollen die Bodenkanten im gleichen Winkel eingebaut werden, den die Schieber zu den Mittelachsen aufweisen, so dass sie parallel zu den Schiebern angeordnet sind. Der Form nach sind Schieber und Bodenkante bevorzugt keilförmig so angeordnet, dass im Vorschub die steile Kante des Schiebers auf die flach ansteigende Seite der Bodenkante trifft und umgekehrt. Sie dichten sich also nicht notwendigerweise gegenseitig ab. Für den Einbau der Bodenkanten bzw. Substratrückhalteleisten ergibt sich in Aufsicht ein Zickzackmuster, wobei die Bodenkanten keine durchgängige Leiste ausbilden, sondern in jedem Falle durch die Mittelachsen unterbrochen werden (siehe Fig. 1, 3, 4, 5).

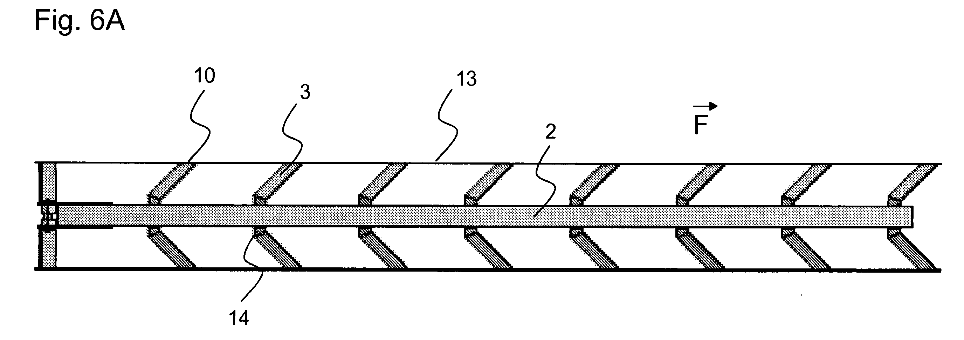

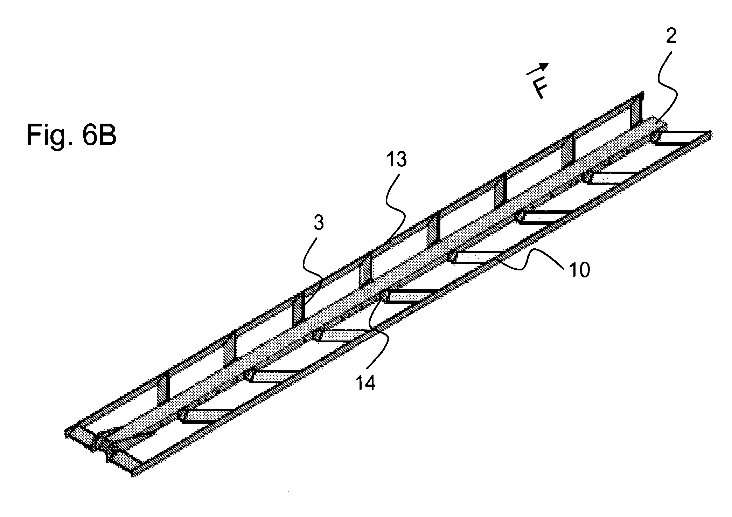

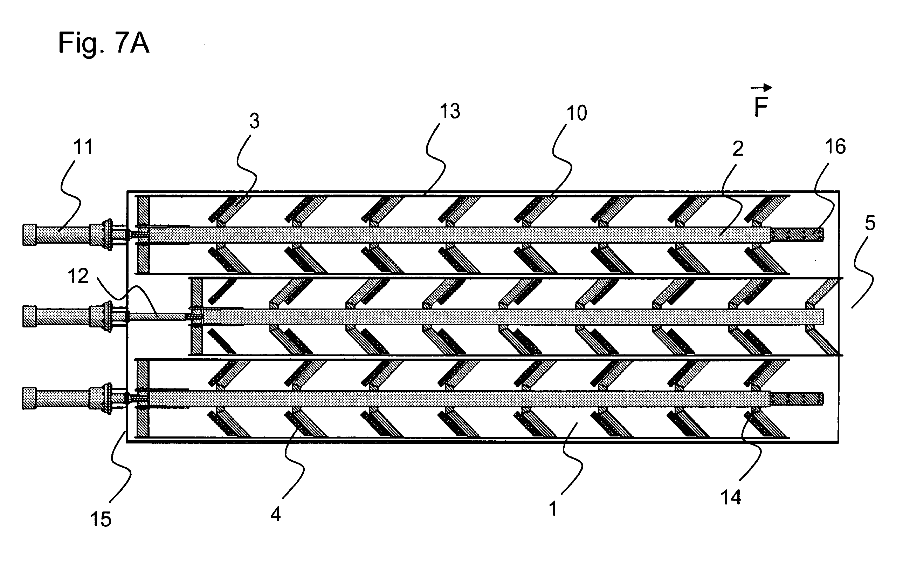

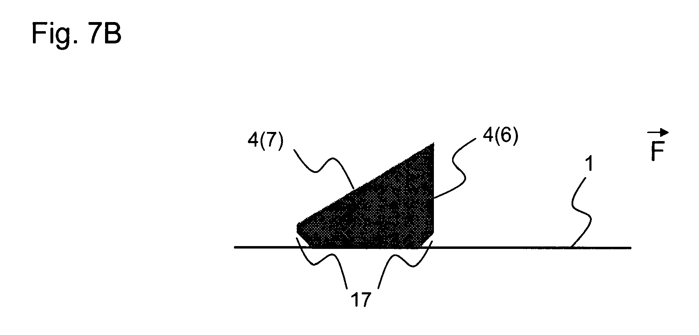

In einer weiteren besonders bevorzugten Ausführungsform werden die Bodenkanten weiterhin an der Schnittstelle zwischen den Schieberarmen zweier benachbarter Mittelachsen unterbrochen, insbesondere wenn die Schieber an ihrem distalen Ende durchgehende Führungsleisten aufweisen (siehe Fig. 6 und 7). Diese Führungsleisten dienen der Stabilisierung der gesamten Schieberkonstruktion. Eine Funktion der Führungsleisten ist auch, dass sie verhindern, dass sich sperrige Fördergutanteile wie z.B. Äste zwischen den Förderarmen verklemmen.

Je nach Ausführungsform können die Bodenkanten aus Kunststoff oder auch aus Metall gefertigt werden. Bodenkanten aus Kunststoff sind besonders geeignet, wenn der Boden des Beschickers ebenfalls aus Kunststoff besteht. Die Bodenkanten werden dann vorzugsweise mit dem Untergrund verschweißt oder geklebt. Bodenkanten aus Metall, die vorzugsweise verschraubt werden, bieten sich insbesondere an, wenn der Boden des Beschickers aus Metall oder Beton besteht.

Nach einer weiteren vorteilhaften Ausführungsform haben die Schieber eine speziell an die Funktion angepasste Form, beispielsweise eine Erhöhung in Förderrichtung und flaches Anliegen entgegen der Förderrichtung, so dass sich ein keilförmiger und/oder dreieckiger Querschnitt ergibt.

Nach einer bevorzugten Ausgestaltung sind die Schieber prismenförmig, so dass sie entgegen der Förderrichtung flach am Boden anliegen und in Förderrichtung eine gewisse Höhe haben, durch die die Menge an Biomasse, die transportiert wird, gröβer ist.

Die Bodenkanten sind in einer vorteilhaften Ausführungsform so ausgestaltet, dass ihre Form ebenfalls die Funktion unterstützt. Dies bedeutet, dass die dem Fördergutauslass zugewandte Seite erhöht ist und steil aufragt und die dem Förderguteintrag zugewandte Seite langsam abfällt, so dass das Fördergut in Art einer sanft ansteigenden Rampe durch die Schieber in Förderrichtung über die Bodenkanten geschoben wird.

Insbesondere wenn die Bodenkanten aus Kunststoff gefertigt werden ist es vorteilhaft, wenn sie an der Oberkante nicht spitz zulaufen, sondern eine Art Leiste ausgebildet wird, die eine größere Stabilität für die Bauteile verleiht. Dies bedeutet, dass die Bodenkanten im Querschnitt nicht dreieckig sind, sondern trapezförmig, wobei die Oberseite die kürzeste Seite darstellt. Eine weitere bevorzugte Ausführungsform für Bodenkanten aus Kunststoff ist derart gestaltet, dass die Bodenkanten mit ihrer Unterseite nicht über ihre gesamte Länge bündig am Boden aufliegen, sondern in und entgegen der Förderrichtung kleine keilförmige Aussparungen aufweisen, die geeignet sind, um die Bodenkanten an diesen Stellen mit dem Boden zu verschweißen, um so eine gute Bodenhaftung zu erreichen. Der Querschnitt durch eine solche Ausführungsform einer Bodenkante ist in Figur 7B dargestellt.

Zwischen Bodenkanten und Schieber befindet sich nach einer Ausführungsform sowohl in Ausgangs- als auch in Endposition der beweglichen Mittelachsen ein kleiner Spalt, so dass diese Teile der Fördereinrichtung nicht bei der Bewegung aneinander reiben. In einer bevorzugten Ausführungsform ist dieser Spalt 1 bis 3 cm breit.

In einer möglichen Ausführungsform sind die Schieber an ihrer hohen, dem Auslass zugewandten Seite beispielsweise 6 bis 8 cm hoch und die entsprechenden Bodenkanten an ihrer hohen Seite 4 bis 6 cm. Generell ist es vorteilhaft, wenn die Bodenkanten von ihrer Dimensionierung her höchstens so hoch wie die Schieber sind, vorzugsweise etwas niedriger. Die zum Boden hinweisende Kante der Bodenkanten ist beispielsweise etwa 6 bis 8 cm lang.

### Kurze Beschreibung der Zeichnungen

Im Folgenden wird die Erfindung noch anhand von Figuren, die bevorzugte Ausführungsformen der Erfindung zeigen, näher erläutert.
Figur 1 zeigt eine Draufsicht auf einen Beschickerboden nach einer Ausführungsform der Erfindung,
Figur 2 zeigt zwei Ansichten von Mittelachsen gemäß einer Ausführungsform der Erfindung, wobei die Mittelachsen einmal von oben (Bildseite oben) und einmal perspektivisch (Bildseite unten) gezeigt sind,
Figur 3 zeigt wieder den Beschickerboden wie die Figur 1, allerdings nur ein Detail und in perspektivischer Ansicht,
Figur 4 zeigt wieder ein Detail aus Figur 1 in noch stärkerer Vergrößerung und horizontal geneigt perspektivischer Ansicht,
Figur 5 zeigt den Beschickerboden gemäß Figur 1 von der Förderguteintragseite aus in perspektivischer Ansicht,
Figur 6A zeigt eine Fördereinrichtung gemäß einer anderen Ausführungsform der Erfindung mit seitlichen Führungsleisten in Draufsicht,
Figur 6B zeigt die Fördereinrichtung der Figur 6A in perspektivischer Sicht,
Figur 7A zeigt eine Draufsicht auf einen Beschickerboden nach der Ausführungsform der Erfindung gemäß den Figuren 6A und 6B mit seitlichen Führungsleisten und
Figur 7B zeigt einen Querschnitt durch eine mögliche Bodenkantenausführung.

### Wege zur Ausführung der Erfindung

Fig.1 zeigt einen möglichen Betriebszustand der Fördereinrichtung in einer Draufsicht. Man sieht auf den Boden 1 in einem Beschicker. Zu erkennen sind drei Mittelachsen 2, an denen jeweils die Schieber 3 in einem Winkel ungleich 90° fixiert sind. Die linke und rechte Mittelachse befindet sich in der Abbildung in ihrer Ausgangsposition, während die mittlere Mittelachse in ihrer Endposition dargestellt ist. Die Förderrichtung ist durch ein F mit Pfeil markiert, der die Richtung von Förderguteintrag zum Fördergutauslass hin anzeigt. Das Fördergut wird in Richtung zum Fördergutauslass 5 hin transportiert. Schwarz eingezeichnet sind die Bodenkanten 4, 4' etc., an denen die Schieber 3 in Ausgangsposition beispielsweise auf der zum Auslass 5 gewandten Seite 6 der Bodenkanten 4 anliegen und in ausgefahrener Position in der vom Auslass 5 abgewandten Seite 7 liegen. Die Bodenkanten 4 sind bevorzugt so gestaltet, dass die Schieber 3 annähernd abdichtend an den Bodenkanten anliegen, wobei kleine Zwischenräume zwischen Schieber und Bodenkanten bleiben können.

Die Positionen 6 und 7 der Bodenkanten in Bezug auf die Schieber sind in der Fig. 1 mit den Bezugszeichen 4(6) und 4(7) markiert. Es sind jeweils die Kanten der Bodenkanten 4 mit der Seite 6 oder 7 gemeint. In Fig. 7B ist dies nochmals erläutert.

Die Gestalt der Schieber 3 ist optimiert dahingehend, dass der Schieber 3 in Förderrichtung der Biomasse eine möglichst hohe Kante hat und beim Zurückfahren möglichst wenig Biomasse wieder zurückschiebt (siehe Fig. 2). Im Betrieb wird der Schieber 3 lediglich zwischen zwei benachbarten Bodenkanten (z.B. 4 und 4' oder 4' und 4") hin und herbewegt.

Fig. 2 zeigt zwei Ansichten einer Mittelachse 2 mit Schieber 3 wie sie aus Fig. 1 bekannt ist, allerdings hier ohne Bodenkante 4, die am Boden des Beschickers aus Fig. 1 angeordnet ist. Oben in der Figur 2 ist die Mittelachse 2 mit den Schiebern 3 von oben her gezeigt, wobei die Förderrichtung wiederum durch den Pfeil F wiedergegeben ist. Auf der unteren Bildseite der Figur 2 ist eine perspektivische Ansicht eines Details aus Figur 1 gezeigt, bei der die dem Fördergutauslass 5 (hier nicht gezeigt) des Beschickerbodens 1 zugewandte und hohe Kante 8 des Schiebers 3 zu erkennen ist. Auf der dem Förderguteintrag zugewandten Seite hat der Schieber 3 eine vergleichsweise flache Kante 9. Zu erkennen ist auch am distalen Ende 10 der Schieber 3 ein trigonaler prismatischer Querschnitt 10 des in dieser Ausführungsform gezeigten Schiebers 3.

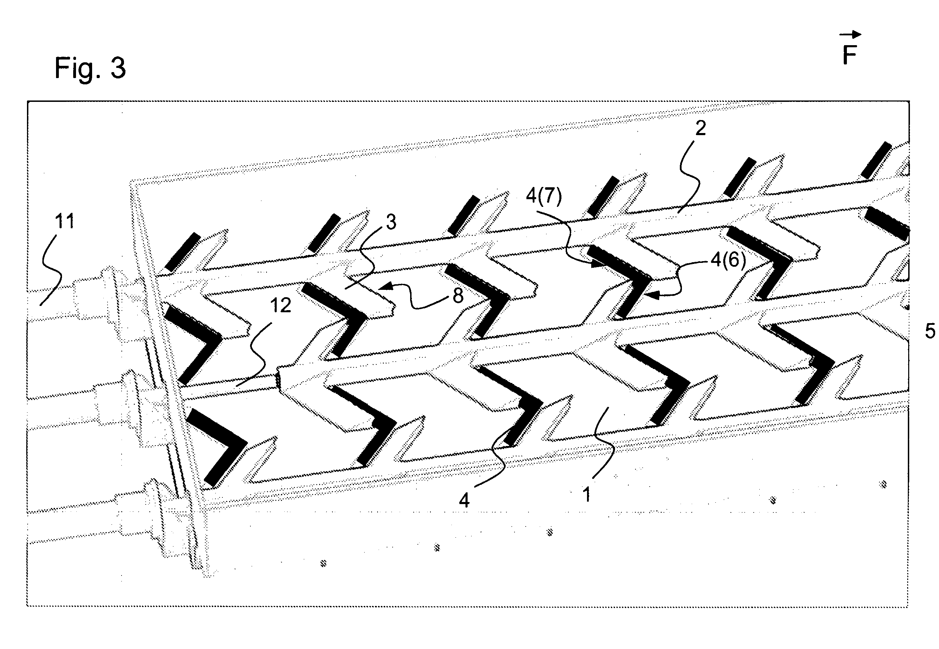

Fig. 3 zeigt eine perspektivischere Ansicht auf einen Boden eines Beschickers 1, wobei der Antrieb 11 der Mittelachsen 2, der über einen Kolbenstange 12 bewerkstelligt wird, zu erkennen ist. Der Kolbenstange 12 ist fest mit der Mittelachse 2 verbunden. Die linke und rechte Mittelachse sind in der Abbildung in ihrer Ausgangsposition gezeigt, die mittlere Mittelachse in ihrer Endposition. Die Fördereinrichtung ist in der Abbildung als Schubboden ausgebildet. Bei einem Zugboden müsste sich der Antrieb auf der gegenüberliegenden Seite befinden.

Die Schieber 3 haben hier eine geringfügig abgeänderte Form ihrer hohen, dem Fördergutauslass 5 zugewandten hohen Kante 8 aus Fig. 2. Zu erkennen ist ein dreieckiger Einschnitt in der Grundseite des Prismas aus Fig. 2, der wiederum zur Steigerung der durch den Schieber 3 aufgenommenen Biomassemenge dient. Im Querschnitt ergibt sich für den Schieber hier die Form einer Pfeilspitze, bei der die Spitze entgegen der Förderrichtung (gekennzeichnet durch F mit Pfeil) zeigt. Die Bodenkanten 4 mit ihrer dem Fördergutauslass 5 zugewandten Seite 4(6) bzw. der entgegen gesetzten Seite 4(7) sind in schwarzer Farbe eingezeichnet.

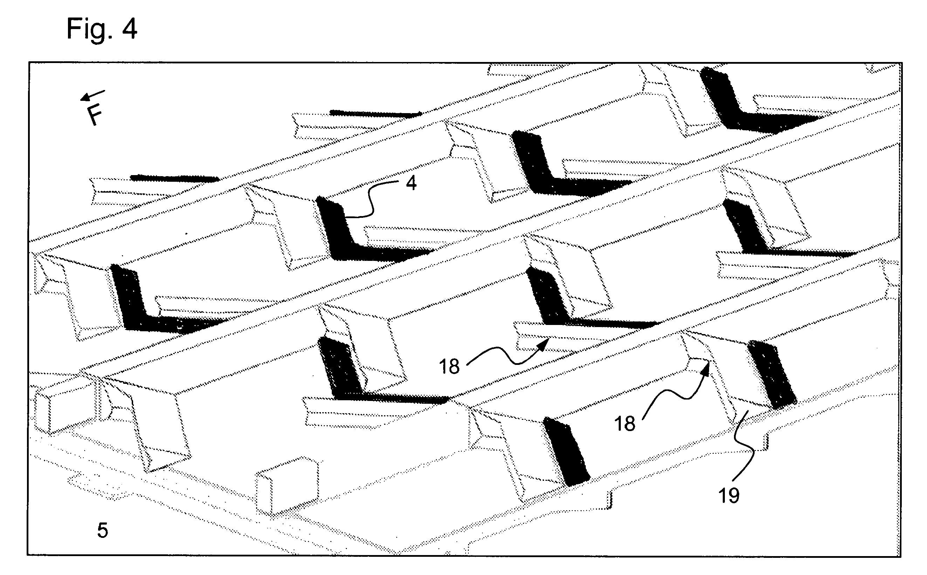

Fig. 4 zeigt die veränderte hohe Kante 8 auf der dem Fördergutauslass 5 zugewandten Seite des Schiebers aus Fig. 3 näher, wobei die der hohen Kante 8 aus Fig. 2 entsprechende Kante hier mit 18 bezeichnet wird. Der pfeilförmige Querschnitt des Schiebers, der sich dadurch ergibt, ist mit dem Bezugszeichen 19 markiert. Die Bodenkanten 4 sind in schwarz eingezeichnet.

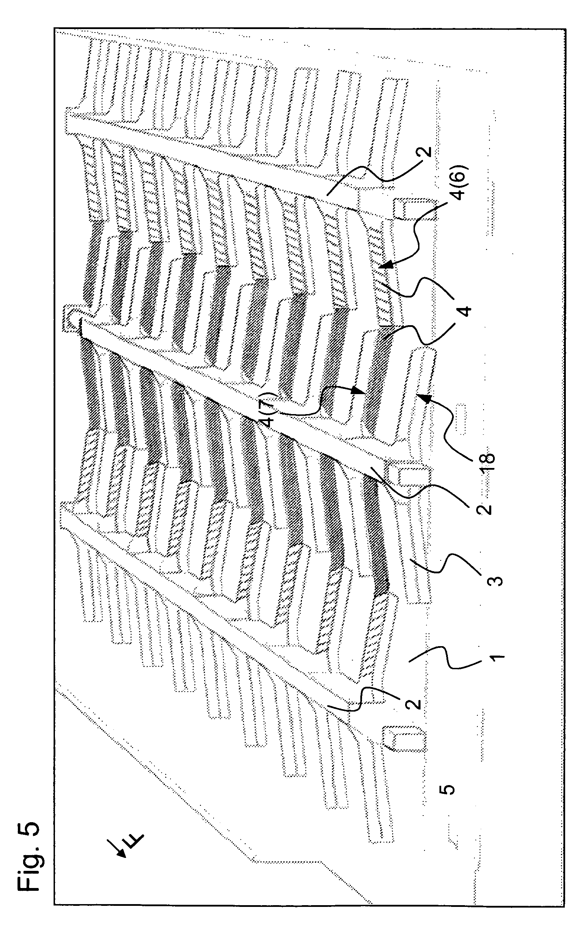

Fig. 5 schließlich zeigt eine perspektivische Ansicht eines Beschickerbodens 1 von der Fördergutauslassseite her. Zu erkennen sind die Schieber 3, die an den Bodenkanten 4 abwechselnd an der Position 6 oder 7 anliegen. Die mittlere Mittelachse 2 befindet sich in der Abbildung in ihrer Endposition. Hier liegen die Schieber auf der dem Fördergutauslass 5 abgewandten Seite der Bodenkanten 4(7)(diese Situation ist dunkelgrau eingezeichnet) an. Die beiden äußeren Mittelachsen 2 befinden sich in der Figur 5 in ihrer Ausgangsposition. Hier liegen die Schieber auf der dem Auslass zugewandten Seite der Bodenkanten 4(6) (diese Situation ist schraffiert eingezeichnet) an. Zu erkennen ist, dass nach dieser bevorzugten Ausführungsform die Bodenkante 4 eine durchgehende Kante zwischen zwei benachbarten Mittelachsen 2 bildet.

Fig. 6 zeigt eine Fördereinrichtung mit seitlichen Führungsleisten 13. Fig. 6A stellt eine Draufsicht auf den Beschickerboden 1 dar, während Fig. 6B eine perspektivische Sicht darstellt. Die Schieber 3 befinden sich in diesem Ausführungsbeispiel in einem Winkel von 45° zur Mittelachse 2. Parallel zu der Mittelachse sind die distalen Enden 10 der Schieber jeweils durch eine Führungsleiste 13 verbunden. Die Schieber 3, die in einem Winkel ungleich 90° zur Mittelachse angeordnet sind, stecken nach einer Ausführungsform in Aufnahmevorrichtungen 14 der Mittelachsen 2, die beispielsweise auch rechtwinklig an der Mittelachse vorgesehen sein können. Die Schieber 3 sind dann in diesen Aufnahmevorrichtungen 14 der Mittelachse 2 fixiert.

Fig. 7A stellt eine Draufsicht auf einen Beschickerboden ähnlich der in Figur 1 gezeigten Ansicht dar. Hier können die Bodenkanten zwischen zwei Mittelachsen nicht durchgehend ausgebildet sein, weil die distalen Enden 10 der Schieber 3 durch Führungsleisten 13, die zwischen zwei benachbarten Bodenkanten 4 angeordnet sind, verbunden sind (siehe Detail in Figur 6).

Fig. 7B zeigt beispielhaft den Querschnitt durch eine mögliche Bodenkantenausführung.

Die Förderrichtung ist in beiden Teilabbildungen wieder durch den Pfeil F markiert.

Fig. 7A zeigt einen Schubboden mit drei Mittelachsen 2, die auf einer am Boden 1 verankerten Schienenunterkonstruktion 16 hin und her gleiten. Die Wand des Beschickers ist mit 15 gekennzeichnet. Der Antrieb erfolgt in Richtung des Fördergutauslasses 5 über drei Hydraulikzylinder 11, deren Kraft jeweils über einen Kolbenstange 12 übertragen wird. Die mittlere Mittelachse befindet sich in der Darstellung in ihrer Endposition, während sich die beiden äußeren Mittelachsen in der Ausgangsposition befinden. Die in einem Winkel ungleich 90 ° angeordneten Schieber 3, sind fest mit den kurzen Aufnahmevorrichtungen 14 der Mittelachsen 2 verbunden. An den distalen Enden 10 sind die Schieberarme 3 über Führungsleisten 13 verbunden. Die Bodenkanten 4 (schwarz eingezeichnet) nehmen jeweils denselben Winkel zur Mittelachse ein wie die Schieberarme 3 und haben in etwa dieselbe Länge wie diese. Die Anordnung der Bodenkanten auf dem Beschickerboden 1 ist durch die Lage der Mittelachsen 2, der Führungsleisten 13 sowie der End- und Grundposition der Förderarme bestimmt, derart dass auch möglicherweise zu allen eben genannten Elementen in jedem Betriebszustand ein kleiner Abstand bestehen bleibt. Die Anzahl der einzeln stehenden Bodenkanten entspricht in dieser Ausführungsform der Anzahl der von der Mittelachse abgehenden Schieberarme.

Fig. 7B zeigt den prismatischen Querschnitt durch eine Bodenkante, wie sie vorzugsweise für Bodenkanten aus Kunststoff verwendet wird. Die in Förderrichtung zeigende Seite der Bodenkante 4(6) ragt steil auf, die gegen die Förderrichtung weisende Seite 4(7) fällt flach ab. Die dem Boden 1 zugewandte Seite weist an ihren Enden zwei Aussparungen 17 auf, die Material aufnehmen können, um die Bodenkante stabil mit dem Boden 1 zu verschweißen.

Die Schieber 3 sind erfindungsgemäß nicht verstellbar, sondern feststehend.

## Patentansprüche

1. Beschicker für Biogasanlagen oder andere Anlagen, die mit Biomasse betrieben werden, umfassend eine Fördereinrichtung, wobei die Fördereinrichtung eine oder mehrere bewegbare Mittelachsen (2) am Boden (1) des Beschickers umfasst, wobei an jeder Mittelachse (2) eine Anzahl feststehender Schieber (3) so angeordnet ist, dass jeder Schieber (3) durch die Bewegung der Mittelachse (2) aus der Anfangsposition an einer ersten Bodenkante (4) heraus bis zu einer Endposition an der nächsten Bodenkante (4') bewegt wird, wobei die Schieber feststehend und symmetrisch auf einander gegenüberliegenden Seiten der Mittelachse (2) angeordnet sind, **dadurch gekennzeichnet, dass** die Schieber in Richtung eines Fördergutauslasses (5) einen Winkel in einem Bereich von 20° bis 80° mit der Mittelachse (2) einschließen.

2. Beschicker nach Anspruch 1, wobei der Schieber (3) prismatisch (8,9,10,18,19) geformt ist.

3. Beschicker nach Anspruch 1 oder 2, wobei der Schieber (3) trigonal prismatisch mit Einschnitt (18,19) geformt ist.

4. Beschicker nach einem der vorstehenden Ansprüche, wobei die Bodenkante (4) in ihrer Lage am Boden (1) des Beschickers und/oder in ihrer Form dem Schieber (3) angepasst ist.

5. Beschicker nach einem der vorstehenden Ansprüche, wobei die eine oder mehrere Mittelachsen von außen angetrieben werden.

6. Beschicker nach einem der vorstehenden Ansprüche, wobei die Bodenkanten (4) aus Kunststoff oder Metall sind.

7. Beschicker nach einem der vorstehenden Ansprüche, wobei die Bodenkanten im Querschnitt prismatisch geformt sind und eine hohe Kante (4(6)) in Förderrichtung und eine flach abfallende Kante (4(7)) entgegen der Förderrichtung haben.

8. Beschicker nach einem der vorstehenden Ansprüche, wobei die Bodenkanten (4) sich von einer Mittelachse zur nächsten Mittelachse erstrecken und in ihrem Verlauf einen Winkel kleiner 180° einschließen.

9. Beschicker nach einem der vorstehenden Ansprüche, wobei die Schieber (3) an ihren distalen Enden (10) durch eine Führungsleiste (13) verbunden sind.

10. Beschicker nach anspruch 9, wobei die Bodenkanten (4) sich von einer Mittelachse bis zur nächsten Führungsleiste (13) erstrecken und in ihrem Verlauf parallel zum Schieber (3) angeordnet sind.

## Claims

1. A feeder for biogas plants or other plants which are operated using biomass, comprising a conveyor, wherein the conveyor comprises one or a plurality of movable central spindles (2) at the base (1) of the feeder, wherein a number of fixed pushers (3) are arranged in such a manner on each central spindle (2) that each pusher (3) is moved by means of the movement of the central spindle (2) out of the initial position at a first base edge (4) to an end position at the next base edge (4'), wherein the pushers are arranged fixedly and symmetrically on mutually opposite sides of the central spindle (2), **characterised in that** the pushers enclose an angle in a range from 20 ° to 80° with the central spindle (2) in the direction of an outlet (5) for conveyed material.

2. The feeder according to Claim 1, wherein the pusher (3) is prismatically (8, 9, 10, 18, 19) shaped.

3. The feeder according to Claim 1 or 2, wherein the pusher (3) is shaped trigonally prismatically with recess (18, 19).

4. The feeder according to one of the preceding claims, wherein the base edge (4) is adapted to the pusher (3) in terms of its position on the base (1) of the feeder and/or in terms of its shape.

5. The feeder according to one of the preceding claims, wherein the one or plurality of central spindles are driven externally.

6. The feeder according to one of the preceding claims, wherein the base edges (4) are made form plastic or metal.

7. The feeder according to one of the preceding claims, wherein the base edges are prismatically shaped in cross section and have a high edge (4 (6)) in the conveying direction and a gently sloping edge (4 (7)) counter to the conveying direction.

8. The feeder according to one of the preceding claims, wherein the base edges (4) extend from one central spindle to the next central spindle and in their course enclose an angle of less than 180°.

9. The feeder according to one of the preceding claims, wherein the pushers (3) are connected at their distal ends (10) by means of a guide strip (13).

10. The feeder according to Claim 9, wherein the base edges (4) extend from a central spindle to the next guide strip (13) and are arranged parallel to the pusher (3) in terms of their course.

## Revendications

1. Dispositif d'alimentation pour des usines de biogaz ou d'autres usines fonctionnant avec une biomasse, comprenant un dispositif de transport, dans lequel le dispositif de transport comprend un ou plusieurs axes médians mobiles (2) au fond (1) du dispositif d'alimentation, dans lequel un certain nombre de poussoirs immobiles (3) sont montés de telle manière sur chaque axe médian (2), que chaque poussoir (3) est déplacé par le mouvement de l'axe médian (2), à partir de la position initiale sur une première arête de fond (4), jusqu'à une position finale sur l'arête de fond (4') suivante, dans lequel les poussoirs sont montés de façon immobile et symétrique sur des côtés opposés de l'axe médians (2), **caractérisé en ce que** les poussoirs forment un angle compris entre 20° et 80° avec l'axe médian (2), dans la direction d'une sortie de produit à transporter (5).

2. Dispositif d'alimentation selon la revendication 1, dans lequel le poussoir (3) est formé de façon prismatique (8, 9, 10, 18, 19).

3. Dispositif d'alimentation selon la revendication 1 ou 2, dans lequel le poussoir (3) est formé de façon trigonale prismatique avec encoche (18, 19).

4. Dispositif d'alimentation selon l'une des revendications précédentes, dans lequel l'arête de fond (4) est adaptée au poussoir (3) en matière de sa position au fond (1) du dispositif d'alimentation dans, et/ou en matière de dans sa forme.

5. Dispositif d'alimentation selon l'une des revendications précédentes, dans lequel les un ou plusieurs axes médians sont actionnés de l'extérieur.

6. Dispositif d'alimentation selon l'une des revendications précédentes, dans lequel les arêtes de fond (4) sont constituées de plastique ou de métal.

7. Dispositif d'alimentation selon l'une des revendications précédentes, dans lequel les arêtes de fond présentent une section transversale prismatique et possèdent une arête haute (4 (6)) dans la direction de transport, et une arête plate inclinée (4 (7)) dans le sens inverse de la direction de transport.

8. Dispositif d'alimentation selon l'une des revendications précédentes, dans lequel les arêtes de fond (4) s'étendent entre un axe médian et l'axe médian suivant, et forment un angle inférieur à 180°dans leur parcours.

9. Dispositif d'alimentation selon l'une des revendications précédentes, dans lequel les poussoirs (3) sont reliés par une baguette de guidage (13) à leurs extrémités distales (10).

10. Dispositif d'alimentation selon la revendication 9, dans lequel les arêtes de fond (4) s'étendent entre un axe médian et la baguette de guidage suivante (13), et sont disposées parallèlement au poussoir (3) dans leur parcours.
